# EUROPEAN PATENT APPLICATION

(11) **EP 2 083 367 A1**
(43) Date of publication of application: **29.07.2009**
(21) Application number: 07792154.2
(22) Date of filing: 08.08.2007
(51) Int. Cl.: G06F 19/00, C12M 1/00, C12N 15/09, C12Q 1/68

(54) **METHOD FOR PREDICTION OF HIGHER-ORDER NUCLEIC ACID STRUCTURE, APPARATUS FOR PREDICTION OF HIGHER-ORDER NUCLEIC ACID STRUCTURE, AND PROGRAM FOR PREDICTION OF HIGHER-ORDER NUCLEIC ACID STRUCTURE**

(30) Priority: 13.11.2006 JP 2006306621
(71) Applicant: Nec Soft, Ltd., Tokyo 136-0082 (JP)
(72) Inventor: AKITOMI, Jou, Koto-ku Tokyo 136-0082 (JP)
(74) Representative: Glawe, Ulrich
(86) International application number: PCT/JP2007/065486
(87) International publication number: WO 2008/059642

(57) **Abstract**

The object of the present invention is to provide a method of predicting a higher-order structure of a nucleic acid sequence typified by a G quartet structure, and an apparatus and a program that execute the method. The method according to the present invention relates to a method of predicting a nucleic acid higher-order structure that predicts a higher-order structure of a nucleic acid sequence, the method, including the steps of: extracting bases capable of forming a higher-order structure as a higher-order structure candidate from said nucleic acid sequence; extracting bases capable of forming a stem structure as a stem structure candidate from said nucleic acid sequence; and searching an optimal combinatorial structure based on the higher-order structure candidate and the stem structure candidate.

SEQUENCE LISTING

<110>  NEC Soft, Ltd.

<120>  A method and apparatus for predicting a higher-order
conformation
        of nucleic acid, and a program for executing said method

<130>  101-04727

<150>  JP2006-306621
<151>  2006-11-13

<160>  2

<170>  PatentIn version 3.4

<210>  1
<211>  21
<212>  DNA
<213>  Artificial

<220>
<223>  Sequence of Figure 3

<400>  1
gggcccggga aagggaaagg g
   21


<210>  2
<211>  21
<212>  DNA
<213>  Artificial

<220>
<223>  Sequence of Figure 4

<400>  2
gggcccggga aagggcccgg g
   21

## Description

### TECHNICAL FIELD

The present invention relates to a method of predicting a nucleic acid higher-order structure, and an apparatus for predicting a nucleic acid higher-order structure and a program for predicting a nucleic acid higher-order structure, the apparatus and the program executing the method of predicting a nucleic acid higher-order structure.

### BACKGROUND ART

It has been known that nucleic acid sequences such as DNA or RNA are composed of four bases called adenine (A), cytosine (C), guanine (G), and thymine (T) or uracil (U) where base pairs are formed based on hydrogen bonds between A and T or U and G and C. These common base pairs are called Watson-Crick base pairs. It is known that nucleic acid sequences form various structures based on combinations of these base pairs. Particularly in functional nucleic acids typified by so-called structural genes such as exons, such structures of sequences have a close connection to the function. A structure of a nucleic acid sequence is mostly composed of Watson-Crick base pairs. However, it has been known that base pairs other than Watson-Crick base pairs can be formed in certain sequences. As used herein, the "base pairs other than Watson-Crick base pairs" typically refers to base pairs other than A-T (U) and G-C (e.g. G-A type base pair), but also includes a higher-order structure other than a general double helix structure, such as a triplex structure (base triple structure) or a quadruplex structure (base quadruple structure) since such a higher-order structure does not correspond to a one-to-one base pair.

As a typical example of such a higher-order structure, a G quartet structure wherein four bases of "G" form a quadruplex can be mentioned. For example, it has been know that, *in vivo,* such a G quartet structure is formed in a telomere sequence present at the end of a eukaryotic chromosome DNA and that the G quartet structure has a function of inhibiting a extension reaction due to a telomerase. There are also a number of reports showing that the G quartet structure is important for binding between a certain target substance and an aptamer molecule, which is an artificial nucleic acid sequence having a specific affinity for the target substance (for example, see Non-Patent Document 1). Accordingly, recognition of the presence of a G quartet structure or any other similar higher-order structure provides a significant clue to identification of an important region for the function of nucleic acid sequences.

However, conventional methods of predicting a nucleic acid structure are not a method of predicting such a higher-order structure since the conventional methods basically use a combination of predictions of Watson-Crick base pairs. Therefore, it has been required to develop a method of predicting a nucleic acid higher-order structure typified by a G quartet structure.
Non-Patent Document 1: Stefan Weiss et al., "RNA Aptamers Specifically Interact with the Prion Protein PrP," Journal of Virology, the November issue, 1997, pp. 8790-8797
Non-Patent Document 2: J. Kondo et al., "Crystal structures of a DNA octaplex with I-motif of G-quartets and its splitting into two quadruplexes suggest a folding mechanism of eight tandem repeats," Nucleic Acids Research, Vol. 32, No. 8, 2004, pp. 2541-2549

### DISCLOSURE OF THE INVENTION

### Problems to be Solved by the Invention

The present invention has been achieved in view of the above-described requirements. An object of the present invention is to provide a method capable of predicting a nucleic acid higher-order structure such as a G quartet structure, and an apparatus and a program that execute the method.

### Means for Solving the Problems

The method of predicting a nucleic acid higher-order structure according to the present invention relates to a method that predicts a higher-order structure of a nucleic acid sequence, the method, including the steps of: extracting bases capable of forming a higher-order structure as a higher-order structure candidate from said nucleic acid sequence; extracting bases capable of forming a stem structure as a stem structure candidate from said nucleic acid sequence; and searching an optimal combinatorial structure based on the higher-order structure candidate and the stem structure candidate.

The apparatus for predicting a nucleic acid higher-order structure according to the present invention relates to an apparatus that predicts a higher-order structure of a nucleic acid sequence, the apparatus, including: a higher-order structure candidate-extracting unit that extracts, from said nucleic acid sequence, bases capable of forming a higher-order structure as a higher-order structure candidate; a stem structure candidate-extracting unit that extracts, from said nucleic acid sequence, bases capable of forming a stem structure as a stem structure candidate; and an optimal structure-searching unit that searches an optimal combinatorial structure, based on the higher-order structure candidate and the stem structure candidate.

The program for predicting a nucleic acid higher-order structure relates to a program that predicts a higher-order structure of a nucleic acid sequence, the program, executing the steps of: extracting bases capable of forming a higher-order structure as a higher-order structure candidate from said nucleic acid sequence; extracting bases capable of forming a stem structure as a stem structure candidate from said nucleic acid sequence; and searching an optimal combinatorial structure based on the higher-order structure candidate and the stem structure candidate.

In addition, in the present invention, the "apparatus for predicting a nucleic acid higher-order structure" and the "program for predicting a nucleic acid higher-order structure" refer to a system and a program that executes the method of predicting a nucleic acid higher-order structure, respectively. As used herein, the term "nucleic acid sequence" refers to sequences of various genes, including DNA and RNA.

### Effect of the Invention

According to the present invention, types of higher-order structures of nucleic acid sequences (e.g. higher-order structures which cannot be predicted by the above-mentioned combination of Watson-Crick base pair predictions.

This is because according to the invention, the stem structure candidate formed by Watson-Crick base pairs and the nucleic acid higher-order structure not derived therefrom are managed using common elements (such as constituent bases or parameters associated with the constituent bases) whereby prediction of a nucleic acid higher-order structure can fall into the scope of secondary structure prediction.

Moreover, the present invention can be utilized as a standard when biologically-significant gene sequences (e.g. critical for control of gene expression) are screened from functionally-unknown nucleic acid sequences.

The reason is as follows. Based on a structure predicted in the conventional secondary structure prediction methods, it is difficult to determine whether or not the original sequence has a certain function. However, nucleic acid higher-order structures that can be predicted by the present invention frequently have certain characteristic function *in vivo.*

Furthermore, according to the present invention, a secondary structure of a nucleic acid sequence capable of having a higher-order structure can be predicted with high accuracy.

The reason is as follows. When conventional secondary structure prediction is performed with respect to a sequence having such a higher-order structure, not only is the higher-order structure obviously unpredictable, but also a region intrinsically capable of forming a higher-order structure may be assigned as that of forming any other structure, so that it is likely to reduce accuracy of prediction with respect to other regions. However, according to the present invention, such a situation hardly occurs.

### BRIEF DESCRIPTION OF THE DRAWINGS

FIG. 1 is a schematic diagram showing configuration of the apparatus for predicting a nucleic acid higher-order structure according to the present invention.
FIG. 2 is a schematic diagram of a stem structure.
FIG. 3 is a flow chart showing operation in the apparatus for predicting a nucleic acid higher-order structure according to the present invention.
FIG 4 is a flow chart showing an example of operation of Step A4.
FIG. 5 is a diagram explaining Example 1.
FIG. 6 is a diagram explaining Example 2.
FIG. 7 is a schematic diagram showing another configuration of the apparatus for predicting a nucleic acid higher-order structure according to the present invention. Description of Reference Symbols

The reference numeral "1" refers to an input device; the reference numeral "2" refers to a data-processing unit; the reference numeral "3" refers to a storage device; the reference numeral "4" refers to an output device; the reference numeral "21" refers to a higher-order structure candidate-extracting unit; the reference numeral "22" refers to a stem structure candidate-extracting unit; the reference numeral "23" refers to an optimal structure-searching unit; the reference numeral "24" refers to an input sequence comparison unit; the reference numeral "31" refers to a structure candidate storage unit.

### BEST MODE FOR CARRYING OUT THE INVENTION

### (Configuration of the apparatus for predicting a nucleic acid higher-order structure according to the present invention, and function of each component included therein)

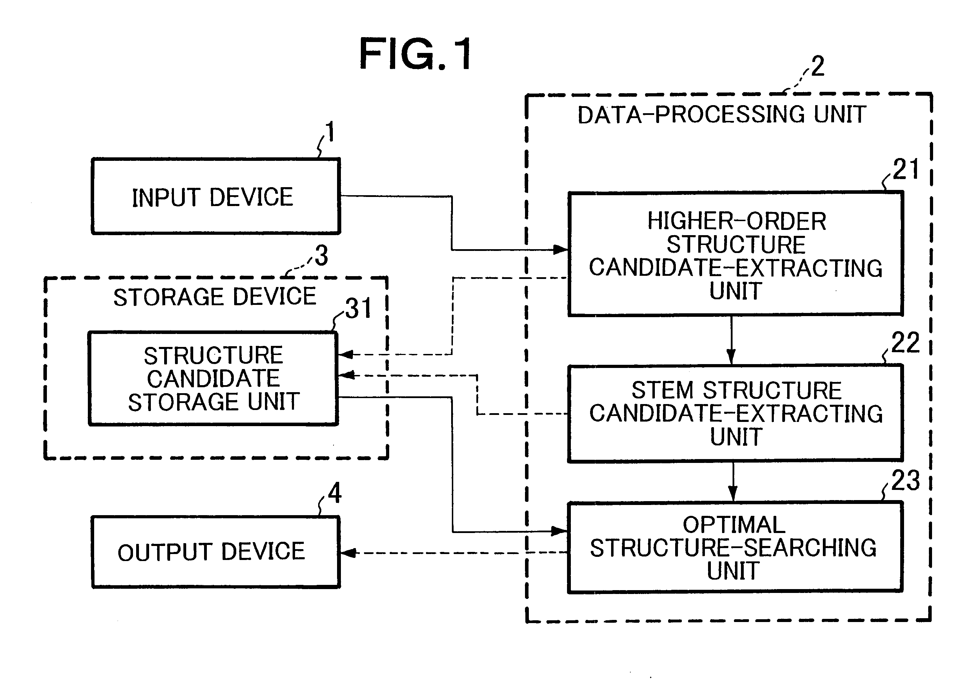

FIG. 1 is a schematic diagram showing the configuration of the apparatus for predicting a nucleic acid higher-order structure according to the present invention. The apparatus for predicting a nucleic acid higher-order structure according to present invention includes an input device 1 such as a keyboard, a data-processing unit 2 operated by program control, a storage device 3 that stores information, and an output device 4 such as a display or a printer.

The data-processing unit 2 includes a higher-order structure candidate-extracting unit 21, a stem structure candidate-extracting unit 22, and an optimal structure-searching unit 23.

The higher-order structure candidate-extracting unit 21 obtains a sequence (that is a target for the structure prediction) inputted through the input device 1, and extracts base combinations capable of forming any higher-order structure from the sequence.

The "any higher-order structure," is not particularly limited as long as the higher structure is a structure that indicates constituent bases in the sequence as a feature of the sequence, e.g. the constituent bases other than Watson-Crick-type base pairs forming the double helix structure. Specifically, it is known that base combinations which are limited to some extent are required in order to form such a structure, and a structure which can indicates a candidate for a region capable of forming such structure in the sequence can be mentioned. Examples of such higher-order structures include a double helix structure formed by base pairs other than Watson-Crick-type base pairs, or a triplex or multiplex structure (such as a triplex structure or quadruplex structure) of the Watson-Crick-type. In particular, a G quartet including four regions of successive guanine residues, or an octaplex structure formed of eight DNA strands gathered together (G quartet, I-motif; see Non-Patent Document 2).

The higher-order structure candidate-extracting unit 21 extracts a higher-order structure candidate from the target sequence for the structure prediction, based on the requirements of bases for the formation of the any higher-order structure. For example, when the higher-order structure, which is a target for the structure prediction, is a G quartet structure, the higher-order structure candidate-extracting unit 21 extracts four regions each including several successive G bases from the sequence. The extracted bases are stored in a structure candidate storage unit 31 as structure candidates. In this case, each stored higher-order structure candidate may optionally have a certain parameter that is an index used for searching of a combinatorial structure by the optimal structure-searching unit 23 with respect to the structure candidates. In this context, for example, the "certain parameter" may be an index indicating how easily the nucleic acid sequence can form the higher-order structure (e.g. a value of the free energy of the structure candidate).

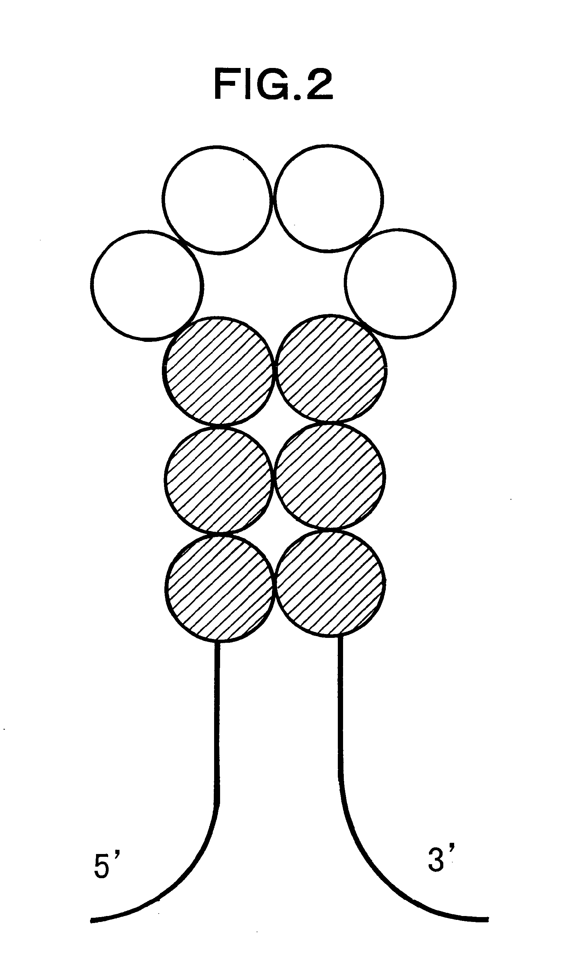

The stem structure candidate-extracting unit 22 extracts a base combination capable of forming a stem structure from the sequence that has subjected to the extraction by the higher-order structure candidate-extracting unit 21. As used herein, the term "stem structure" refers to a region including successive Watson-Crick base pairs (e.g. the structure of the portion indicated by shaded circles in FIG 2). Similarly to the higher-order structure candidate, the stem structure may optionally have a certain parameter which is an index used for searching of a combinatorial structure by the optimal structure-searching unit 23 with respect to the structure candidates. The extracted bases are stored as structure candidates in the structure candidate storage unit 31 together with the structure candidates stored through the higher-order structure candidate-extracting unit 21. The "certain parameter" which is an index used for searching of a combinatorial structure may be the same as the parameter as for the higher-order structure candidate-extracting unit 21.

The optimal structure-searching unit 23 performs searching of an optimal combinatorial structure (herein also referred to as "combinatorial structure search") based on the higher-order structure candidates and the stem structure candidates stored in the structure candidate storage unit 31. In this case, an algorithm for the combinatorial structure search is at least required to eliminate any contradiction such as an overlap among the bases used in the higher-order structure candidates and the stem structure candidates, respectively. Examples of the algorithm may include all sorts of methods such as a method of selecting a combination of structure candidates that minimizes the free energy of the whole structure, a method of selecting a structure candidate with a neural network, and a method of selecting a structure candidate with a genetic algorithm. Thus, any algorism meeting the demand of the user may be selected.

The storage device 3 includes the structure candidate storage unit 31.

The structure candidate storage unit 31 stores information such as positions of bases necessary to form the structure candidate, which is extracted by the higher-order structure candidate-extracting unit 21 or by the stem structure candidate-extracting unit 22. The information stored therein includes a certain parameter such as a value of the free energy of each structure candidate at the time of formation of the structure, the value to be used in the optimal structure-searching unit 23.

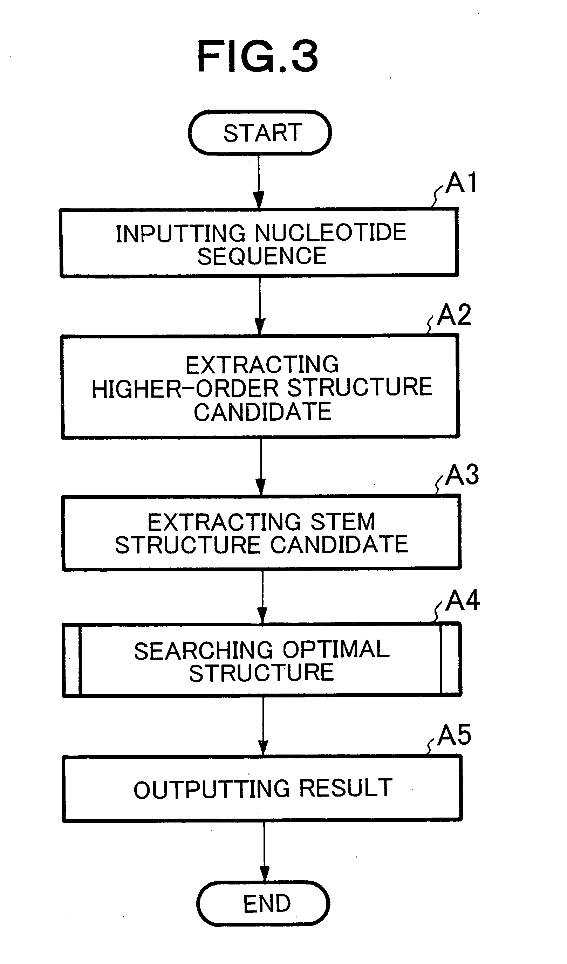

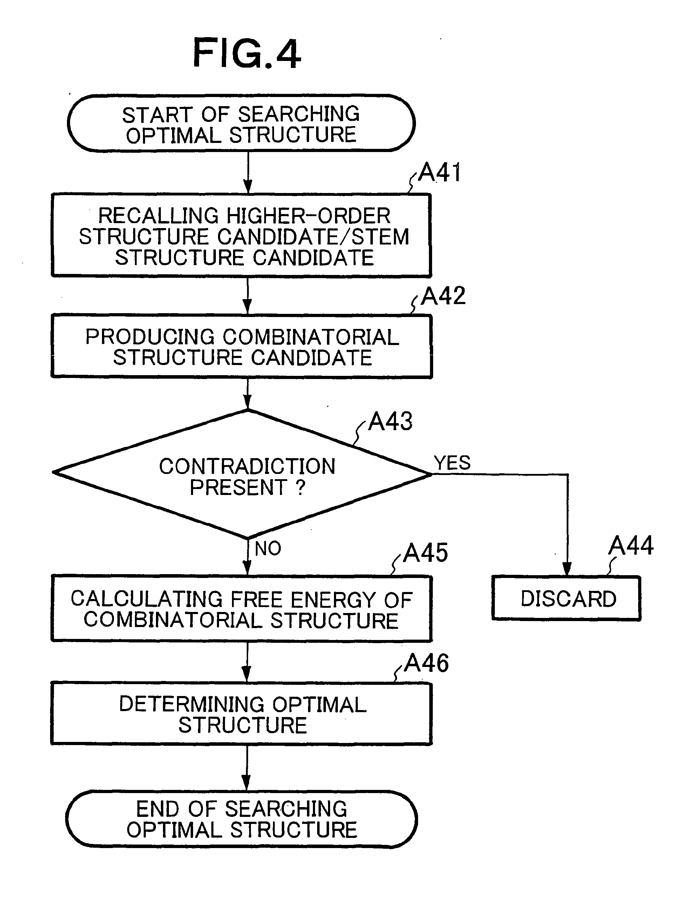

(Each Step of the Method of Predicting Nucleic Acid Higher-Order Structure according to the Present Invention, and Operation of the Apparatus for Predicting Nucleic Acid Higher-Order Structure and the Program for Predicting Nucleic Acid Higher-Order Structure according to the Present Invention)
Hereinafter, with reference to FIG. 1 and the flow charts of FIGS. 3 and 4, each step of the method of predicting a nucleic acid higher-order structure according to the present invention, and operation of the apparatus for predicting a nucleic acid higher-order structure and the program for predicting a nucleic acid higher-order structure according to the present invention will be described in detail.

At first, a nucleic acid sequence provided using the input device 1 is transmitted to the higher-order structure candidate-extracting unit 21 (A1).

From the transmitted sequence, the higher-order structure candidate-extracting unit 21 extracts a characteristic base combination that satisfies requirements for formation of any higher-order structure. For example, when a G quartet structure is a target for prediction of any higher-order structure, the higher-order structure candidate-extracting unit 21 extracts four regions each including several successive bases of "G". A suitable parameter is assigned to the extracted higher-order structure candidate, depending on the constituent bases. The suitable parameter corresponds to the "certain parameter" as described above for the higher-order structure candidate-extracting unit 21. For example, the suitable parameter may be an index indicating how easily the provided sequence can form the higher-order structure to be predicted (e.g. a value of the free energy of the higher-order structure candidate). The information of the higher-order structure candidate which has been assigned the parameter is stored in the structure candidate storage unit 31 together with the information of the nucleotide sequence of the extracted higher-order structure candidate. This procedure is repeated as long as any other combination of bases capable of forming the higher-order structure can be found in the input sequence (A2).

Then, the nucleic acid sequence, which is subjected to extraction by the higher-order structure candidate-extracting unit 21, is transmitted to the stem structure candidate-extracting unit 22. From the sequence, the stem structure candidate-extracting unit 22 extracts a base combination capable of forming a stem structure, and assigns a suitable parameter thereto, depending on the constituent bases. Similarly to the higher-order structure candidate, the information of the stem structure candidate extracted in this manner is also stored in the structure candidate storage unit 31 together with the information of the nucleotide sequence of the extracted stem structure. This procedure is also repeated as long as any other combination of bases capable of forming a stem structure can be found in the input sequence (A3).

Then, based on the information of the higher-order structure candidates and the stem structure candidates stored in the structure candidate storage unit 31, the optimal structure-searching unit 23 searches an combinatorial structure optimal for a secondary structure of the nucleic acid sequence among combinatorial structure candidates obtained by singularly or randomly combining the stored higher-order structure candidates and/or the stored stem structure candidates, such that any contradiction such as an overlap among the bases of the structure candidates is not present. An example of the step of searching such an optimal combinatorial structure will be described with reference to FIG. 4.

FIG. 4 is a flow chart showing an example of operation in Step A4. In Step A4, the optimal structure-searching unit 23 recalls the information of the higher-order structure candidates and the stem structure candidates stored in the structure candidate storage unit 31 (A41). Then, the optimal structure-searching unit 23 generates a combinatorial structure candidate obtained by singularly combining the higher-order structure candidates or the stem structure candidates, or by combining both the higher-order structure candidates and the stem structure candidates (A42). The optimal structure-searching unit 23 then determines whether or not each combinatorial structure candidate has a contradiction such as an overlap among the bases of the candidate (A43). If it is determined in Step A43 that a contradiction is present, the optimal structure-searching unit 23 discards the corresponding combinatorial structure candidate (A44). If it is determined in Step A43 that no contradiction is present, the optimal structure-searching unit 23 retains the corresponding combinatorial structure candidate as the combinatorial structure of the present invention, and calculates the free energy of the corresponding combinatorial structure by summing the free energies of the higher-order structure candidate(s) and/or the stem structure candidate(s) that forms the combinatorial structure candidate (A45). Based on the free energies each calculated as described above, the optimal structure-searching unit 23 then determines which combinatorial structure is optimal for the structure formed by the nucleic acid sequence (A46). This determination may a method of determining a combinatorial structure giving the lowest free energy as the optimal structure. Thus, the step of searching an optimal combinatorial structure is completed.

The step of searching an optimal combinatorial structure may be modified by any other method using a neural network, a genetic algorithm or the like (A4).

Then, in Step A5, the data-processing unit 2 outputs, to the output device 4, the information about the combinatorial structure that is determined as the optimal structure in the optimal structure-searching unit 23. When outputting the information, the data-processing unit 2 may also output, to the output device 4, information of whether or not the optimal structure determined in the optical structure- searching unit 23 includes the higher-order structure candidate extracted in the higher-order structure candidate-extracting unit 21. Furthermore, the data-processing unit 2 may also output the information about combinatorial structures other than the combinatorial structure determined as the optimal structure in the optimal structure-searching unit 23, together with the above-mentioned information of suitable parameters.

### (Other Components)

In the present invention, a plurality of nucleic acid sequences may be the target for the structure prediction. In this case, a certain relationship between the plurality of nucleic acid sequences may be used as an index for the combinatorial structure search in combination with the parameter such as the free energy. As examples of the plurality of nucleic acid sequences, plural evolutionarily-conserved sequences, plural sequences conserved among species, plural sequences having a similar function, or the like can be mentioned. When the plurality of nucleic acid sequences are used, the above-mentioned index of a certain relationship between the plurality of nucleic acid sequences may be the degree of conservation between sequences, the homology between sequences, or a similarity in appearance frequency of bases extracted using a count vector or the like.

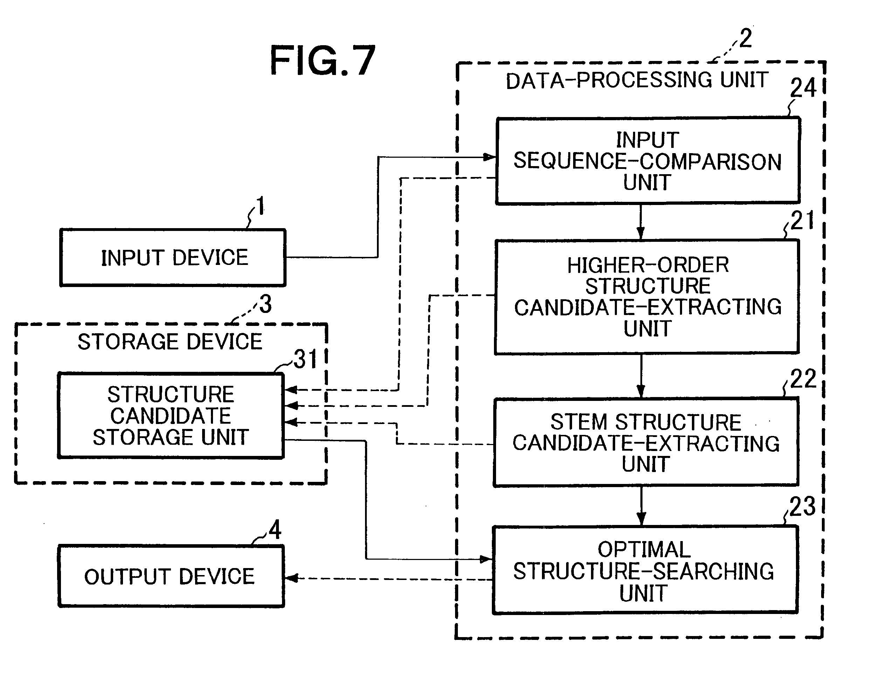

An example where a plurality of nucleic acid sequences are assigned as the target for the structure prediction will be described with reference to FIG. 7. FIG. 7 is a schematic diagram showing another configuration of the apparatus for predicting a nucleic acid higher-order structure according to the present invention. As shown in FIG. 7, the apparatus for predicting a nucleic acid higher-order structure according to the present invention includes an input device 1, a data-processing unit 2, a storage device 3, and an output device 4. The data-processing unit 2 includes a higher-order structure candidate-extracting unit 21, a stem structure candidate-extracting unit 22, an optimal structure-searching unit 23, and an input sequence comparison unit 24. The configuration and operation of the input device 1, the data-processing unit 2, the output device 4, the higher-order structure candidate-extracting unit 21, the stem structure candidate-extracting unit 22, and the optimal structure-searching unit 23 are the same as described above. Therefore, while the description thereof will be omitted, the configuration, function, and operation of the input sequence comparison unit 24 will be mainly described below.

The function of the input sequence comparison unit 24 will be described. With regard to a plurality of nucleic acid sequences inputted from the input device 1, the input sequence comparison unit 24 quantifies a certain relationship between the plurality of nucleic acid sequences. The certain relationship may be quantified using a method of searching a homology such as alignment, a method of comparing the appearance frequencies of bases extracted using a count vector, or the like. The numerical value derived from the certain relationship may be the degree of conservation or homology between the respective sequences described above as the index. The resulting index is stored in the structure candidate storage unit 31, and then, is used for the combinatorial structure search by the optimal structure-searching unit 23 (A45).

Hereinafter, each step in the method of predicting a nucleic acid higher-order structure according to the present invention, and the operation of the apparatus for predicting a nucleic acid higher-order structure and the program for predicting a nucleic acid higher-order structure according to the present invention wherein the input sequence comparison unit 24 is provided will be described.

A plurality of nucleic acid sequences is inputted from the input device 1, and then, Steps A2 to A3 and Steps A41 to A43 are performed. In Step A45, the optimal structure-searching unit 23 sums the free energies retained in the higher-order structure candidate(s) and/or the stem structure candidate(s) forming the combinatorial structure, which is determined to have no contradiction, thereby being retained in Step A43, whereby the free energy of the combinatorial structure is calculated. Simultaneously, the optimal structure-searching unit 23 weights the calculated free energy with the index calculated by the input sequence comparison unit 24. Based on each free energy weighted as described above, the optimal structure-searching unit 23 determines which combinatorial structure is optimal for the structure formed by each nucleic acid sequence (A46). Thereafter, the information about the combinatorial structure, etc. is outputted in Step A5.

By using the input sequence comparison unit in the above-described manner, prediction can be performed with high accuracy with respect to each sequence, depending on the similarity between the plurality of input sequences.

In FIGS. 1 and 7, the arrow lines from other devices to the data-processing unit 2 are drawn by solid lines while the arrow lines from the data-processing unit 2 to other devices are drawn by dotted lines.

### EXAMPLES

Hereinafter, the operation will be specifically described with reference to some examples.

### (Example 1)

Input Sequence 1 (GGGCCCGGGAAAGGGAAAGGG, SEQ ID NO: 1) is given as the input nucleic acid sequence through the input device 1. Then, it is predicted whether or not the sequence includes a G quartet structure (A1).

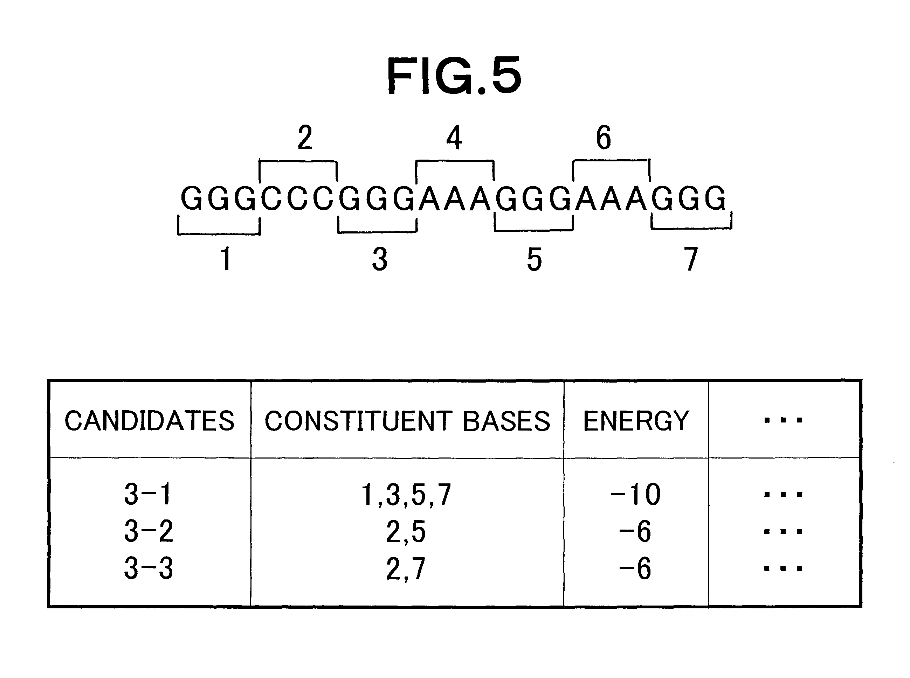

The higher-order structure candidate-extracting unit 21 extracts characteristic base combinations necessary to form any higher-order structure from Input Sequence 1. In this example, the "any higher-order structure" refers to a G quartet structure. Therefore, four regions each including successive bases of G are extracted from Input Sequence 1. When the length of the successive region is set to 3 or more and the free energy due to stacking in a G quartet structure formed by three G quartet planes is set to -10, the higher-order structure candidate-extracting unit 21 stores Candidate 3-1 shown in FIG. 5 in the structure candidate storage unit 31 as a higher-order structure candidate. No higher-order structure candidate other than Candidate 3-1 can be extracted from Input Sequence 1 under the conditions set forth above (A2).

Then, the stem structure candidate-extracting unit 22 extracts base combinations necessary to form a stem structure from Input Sequence 1. When the length of the successive base region is set to 3 or more in the same manner as the higher-order structure candidate-extracting unit 21 and the free energy of stacking between G-C/G-C base pairs is assumed to be -3, the stem structure candidate-extracting unit 22 stores Candidates 3-2 and 3-3 shown in FIG. 5 in the structure candidate storage unit 31 as stem structure candidates in the same manner as the above Candidate 3-1. No stem structure candidate other than Candidates 3-2 and 3-3 can be extracted from Input Sequence 1 (A3).

Then, the optimal structure-searching unit 23 performs the step of searching an optimal combinatorial structure. At first, the optimal structure-searching unit 23 recalls Candidate 3-1 (i.e. a higher-order structure candidate) and Candidates 3-2 and 3-3 (i.e. stem structure candidates) stored in the structure candidate storage unit 31 (A41). Then, the optimal structure-searching unit 23 randomly combines Candidates 3-1 to 3-3 to generate combinatorial structure candidates (A42), and determines whether they have a contradiction (A43). In this case, the constituent bases of Candidates 3-1, 3-2 and 3-3 of FIG. 5 contradict with one another. Therefore, the optimal structure-searching unit 23 retains each of Candidates 3-1 to 3-3 from Input Sequence 1 as a combinatorial structure. If the energy of the loop structure formed simultaneously with the formation of these candidates is neglected, the energy of the structure formed by Input Sequence 1 is equal to the sum of the free energies of the respective structure candidates. Therefore, the optimal structure-searching unit 23 calculates the sum of the free energies with respect to Candidates 3-1 to 3-3 based on the above-set values of the free energy (A45). As a result, the sum of free energies with respect to Candidates 3-1 to 3-3 is calculated as -10, -6 and -6, respectively (see FIG. 5). Based on the calculated free energies obtained in this manner, the optimal structure-searching unit 23 then determines which combinatorial structure the nucleic acid sequence can optimally have (A46). In this case, the structure formed by Candidate 3-1 is a stable structure with the lowest free energy value. Therefore, the result of the step of searching an optimal combinatorial structure by way of minimizing the free energy is Candidate 3-1 alone (A46). The result is outputted through the output device 4 (A5). Since Candidate 3-1 is a candidate for a G quartet structure, the result of the prediction that Input Sequence 1 can have a higher-order structure of a G quartet structure is obtained.

### (Example 2)

In the above-described manner, Input Sequence 2 (GGGCCCGGGAAAGGGCCCGGG, SEQ ID NO: 2), which is obtained by modifying parts of Input Sequence 1, is given through the input device 1 as the input nucleic acid sequence. It is predicted whether the sequence includes a G quartet structure (A1).

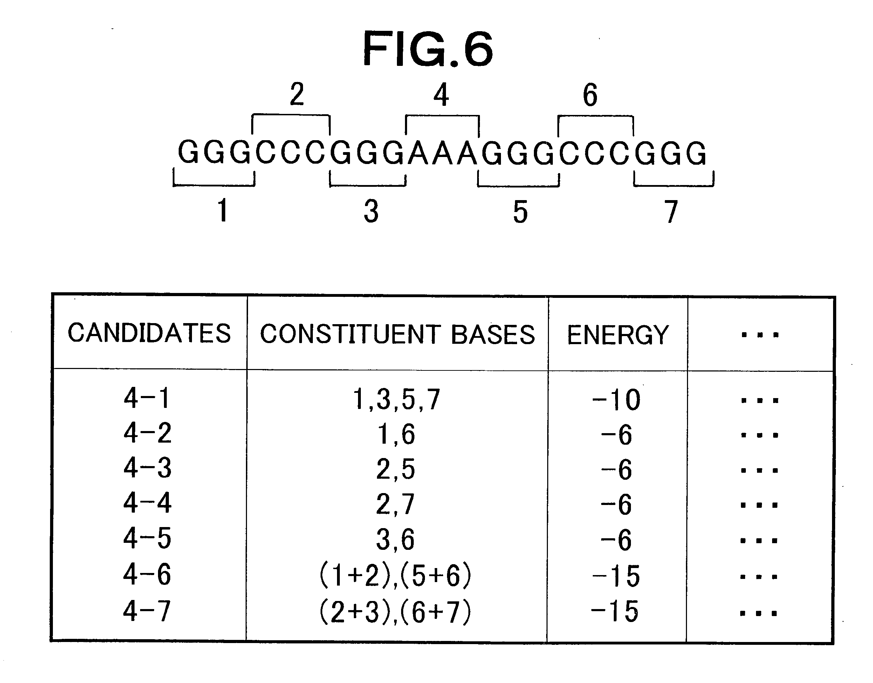

In the same manner as Example 1, the higher-order structure-extracting unit 21 is used to extract higher-order structure candidates from Input Sequence 2, and the stem structure candidate-extracting unit 22 is used to extract stem structure candidates from Input Sequence 2 (A2, A3). In the same manner as Example 1, when the length of the successive region is set to 3 or more, the free energy due to stacking in a G quartet structure formed by three G quartet planes is set to -10, and the free energy due to stacking between G-C/C-G base pairs is also assumed to be -3, the structure candidate information shown in FIG. 6 is stored in the structure candidate storage unit 31 (A2, A3).

Then, the optimal structure-searching unit 23 performs the step of searching an optimal combinatorial structure. At first, the optimal structure-searching unit 23 recalls Candidate 4-1 (i.e. a higher-order structure candidate) and Candidates 4-2 to 4-5 (i.e. stem structure candidates) stored in the structure candidate storage unit 31 (A41). Then, the optimal structure-searching unit 23 randomly combines Candidates 4-1 to 4-5 to generate combinatorial structure candidates (A42), and determines whether they have a contradiction (A43). In this case, the combination of Candidates 4-2 and 4-3 has no contradiction while the combination is equal to Candidate 4-6. In the same manner as Candidates 4-2 and 4-3 and Candidate 4-6, the combination of Candidates 4-4 and 4-5 has no contradiction while the combination is equal to Candidate 4-7. Therefore, the optimal structure-searching unit 23 retains each of Candidates 4-1, 4-6 and 4-7 from Input Sequence 2 as a combinatorial structure. Based on the same consideration in the case of Input Sequence 1 shown in Example 1, the optimal structure-searching unit 23 calculates the sum of the free energies with respect to Candidates 4-1, 4-6 and 4-7 based on the above-set values of the free energy (A45). As a result, the sum of the free energies with respect to Candidates 4-1, 4-6 and 4-7 is calculated as -10, -15 and -15, respectively (see FIG. 6). Based on the calculated free energies, the optimal structure-searching unit 23 then determines which combinatorial structure the nucleic acid sequence can optimally have (A46). In this case, a structure formed by either Candidate 4-6 or 4-7 is a stable structure with the lowest free energy value. Therefore, the result of the step of searching an optimal combinatorial structure by way of minimizing free energy is any one of Candidates 4-6 and 4-7 (A46). The result is outputted through the output device 4 (A5). Since neither Candidate 4-6 nor Candidate 4-7 has a G quartet structure, the result of the prediction that Input Sequence 2 cannot have any higher-order structure of a G quartet structure can be obtained.

### INDUSTRIAL APPLICABILITY

In recent post-genome research, it has been discovered that functional nucleic acids (in particular, ncRNAs) play various important *in vivo* functions such as gene expression control, and some ncRNAs have been identified. Therefore, more detailed experiments have to be carried out to understand how they actually function *in vivo.* However, techniques of informatic screening of ncRNAs to be subjected to such experiments are still scarce. Concerning such a problem, it is estimated that some sequences having a higher-order structure predictable according to the present invention have a certain function. Accordingly, the present invention can be applied to such screening of ncRNAs.

Furthermore, it is also estimated that such a higher-order structure have an important function in an aptamer molecule, which is an artificial nucleic acid sequence. Accordingly, when a certain nucleic acid sequence that forms an aptamer molecule is given, the present invention can also be applied to identification of a region important for binding between the aptamer molecule and a target substance with respect to the sequence.

[0051] The present invention has been described with preferred embodiments thereof. While the present invention has been described with reference to specific examples, it will be understood that various modifications and changes may be made to the specific examples without departing from the spirit and scope of the claimed invention. Accordingly, the scope of the invention should not be interpreted as being limited to the detailed description and the attached drawings.

## Claims

1. A method of predicting a nucleic acid higher-order structure that predicts a higher-order structure of a nucleic acid sequence, the method, comprising the steps of:
extracting bases capable of forming a higher-order structure as a higher-order structure candidate from said nucleic acid sequence;
extracting bases capable of forming a stem structure as a stem structure candidate from said nucleic acid sequence; and
searching an optimal combinatorial structure based on the higher-order structure candidate and the stem structure candidate.

2. The method of predicting a nucleic acid higher-order structure according to Claim 1, wherein the step of searching the optimal combinatorial structure is a step in which, among combinatorial structures obtained by singularly or randomly combining the higher-order structure candidate and/or the stem structure candidate, a combinatorial structure giving a minimum free energy is assigned as said optimal combinatorial structure where no contradiction is present among bases of the higher-order structure candidate and the stem structure candidate.

3. The method of predicting a nucleic acid higher-order structure according to Claim 1 or 2, further comprising the step of quantifying a relationship between a plurality of nucleic acid sequences, wherein the plurality of nucleic acid sequences is said nucleic acid sequence

4. The method of predicting a nucleic acid higher-order structure according to Claim 3, wherein the step of quantifying the relationship between the plurality of nucleic acid sequences is a step in which a degree of conservation between the plurality of nucleic acid sequences is calculated.

5. The method of predicting a nucleic acid higher-order structure according to Claim 3 or 4, wherein the plurality of nucleic acid sequences is evolutionarily conserved.

6. The method of predicting a nucleic acid higher-order structure according to any one of Claims 1 to 5, wherein the higher-order structure is a G quartet.

7. An apparatus for predicting a nucleic acid higher-order structure that predicts a higher-order structure of a nucleic acid sequence, the apparatus, comprising:
a higher-order structure candidate-extracting unit that extracts, from said nucleic acid sequence, bases capable of forming a higher-order structure as a higher-order structure candidate;
a stem structure candidate-extracting unit that extracts, from said nucleic acid sequence, bases capable of forming a stem structure as a stem structure candidate; and
an optimal structure-searching unit that searches an optimal combinatorial structure, based on the higher-order structure candidate and the stem structure candidate.

8. The apparatus for predicting a nucleic acid higher-order structure according to Claim 7, wherein, among combinatorial structures obtained by singularly or randomly combining the higher-order structure candidate and/or the stem structure candidate, the optimal structure-searching unit assigns a combinatorial structure giving the lowest free energy as said optimal structure where no contradiction is present among bases of the higher-order structure candidate and the stem structure candidate

9. The apparatus for predicting a nucleic acid higher-order structure according to Claim 7 or 8, further comprising an input sequence comparison unit that quantifies a relationship between a plurality of nucleic acid sequences, wherein the plurality of nucleic acid sequences is said nucleic acid sequence.

10. The apparatus for predicting a nucleic acid higher-order structure according to Claim 9, wherein the input sequence comparison unit calculates a degree of conservation between the plurality of nucleic acid sequences.

11. The apparatus for predicting a nucleic acid higher-order structure according to Claim 9 or 10, wherein the plurality of nucleic acid sequences is evolutionarily conserved.

12. The apparatus for predicting a nucleic acid higher-order structure according to any one of Claims 7 to 11, wherein the higher-order structure is a G quartet.

13. A program for predicting a nucleic acid higher-order structure that predicts a higher-order structure of a nucleic acid sequence, the program, executing the steps of:
extracting bases capable of forming a higher-order structure as a higher-order structure candidate from said nucleic acid sequence;
extracting bases capable of forming a stem structure as a stem structure candidate from said nucleic acid sequence; and
searching an optimal combinatorial structure based on the higher-order structure candidate and the stem structure candidate.

14. The program for predicting a nucleic acid higher-order structure according to Claim 13, wherein the step of searching the optimal combinatorial structure is a step in which, among combinatorial structures obtained by singularly or randomly combining the higher-order structure candidate and/or the stem structure candidate, a combinatorial structure giving a minimum free energy is assigned as said optimal combinatorial structure where no contradiction is present among bases of the higher-order structure candidate and the stem structure candidate.

15. The program for predicting a nucleic acid higher-order structure according to Claim 13 or 14, further executing the step of quantifying a relationship between a plurality of nucleic acid sequences, wherein the plurality of nucleic acid sequences is said nucleic acid sequence.

16. The program for predicting a nucleic acid higher-order structure according to Claim 15, wherein the step of quantifying the relationship between the plurality of nucleic acid sequences is a step in which a degree of conservation between the plurality of nucleic acid sequences is calculated.

17. The program for predicting a nucleic acid higher-order structure according to Claim 15 or 16, wherein the plurality of nucleic acid sequences is evolutionarily conserved.

18. The program for predicting a nucleic acid higher-order structure according to any one of Claims 13 to 17, wherein the higher-order structure is a G quartet.
